# EUROPEAN PATENT APPLICATION

(11) **EP 2 431 471 A1**
(43) Date of publication of application: **21.03.2012**
(21) Application number: 10177265.5
(22) Date of filing: 17.09.2010
(51) Int. Cl.: C12N 15/82, C12N 9/14

(54) **Methods for increasing the size of plants or plant organs**

(71) Applicant: Université Catholique de Louvain, 1348 Louvain-La-Neuve (BE)
(72) Inventor: Boutry, Marc, 1150 Bruxelles (BE); Duby, Geoffrey, 5081 Meux (BE); Piotrowiak, Dominik, 64-130 Rydzyna (PL)
(74) Representative: Vanhalst, Koen

(57) **Abstract**

The present invention relates to methods for the production of transgenic plants having increased size or comprising one or more organs or tissues having increased size through expressing heterologous H⁺-ATPase in plants.

## Description

### FIELD OF THE INVENTION

The present invention relates to methods for the production of transgenic plants having increased size and/or containing one or more organs or tissues with increased size. The present invention further discloses the genetic constructs and vectors of these methods and their use, as well as the transgenic plants obtained by the methods of the present invention and their harvestable parts and propagation material.

### BACKGROUND OF THE INVENTION

The plant plasma membrane H⁺-ATPase (PMA) couples hydrolysis of ATP with proton transport outside the cell. This proton-motive force is then used by a number of secondary transporters, which couple proton transport, using symport or antiport, with transport of various ions and metabolites. The plant PMA is associated with many physiological processes related to transport in plants.

WO99/07863 describes processes for increasing the yield in plants comprising stably integrating a nucleotide sequence encoding a PMA from *Solanum tuberosum* or from *Saccharomyces cerevisiae* together with a sucrose transporter into the genome of plants. The nucleotide sequence is put under the control of a promoter that allows transcription specifically in the companion cells, resulting in improved sucrose loading in the phloem and thus in an overall increase of the biomass.

However, over-expression of two *PMA* genes from *Nicotiana plumbaginifolia, PMA4* or *PMA2,* in *Nicotiana tabacum,* making use of an enhanced promoter (2ENpPMA4), resulted in increased PMA expression but did not show any particular phenotype, nor any increase in the measured ATPase activity *in vivo,* suggesting that post-transcriptional down-regulation (for instance, at the enzyme level) compensates over-expression. In contrast, a truncated *PMA4 form,* which is constitutively activated (Zhao et al., 2000, Plant Cell 12, 535-546; Gévaudant et al., 2007, Plant Physiol 144, 1763-1776), was successfully over-expressed in *Nicotiana tabacum* and these plants were affected at various levels: twisted stems, upside-down leaves, male sterility and reduced sensitivity to salt stress, but no increase of organ size was observed (Gévaudant et al., 2007, Plant Physiol 144, 1763-1776; Merlot et al., 2007, EMBO J 26, 3216-3226).

The problem underlying the present invention is to provide further methods for increasing the yield in plants, more particularly in specific plant tissues or organs, such as for example roots, flowers, fruits, seeds, flowers, by expressing (including over-expressing) a PMA in said plants.

The problem is solved according to the invention by producing a transgenic plant expressing an exogenous PMA from another plant species as provided by the embodiments characterized in the claims.

### SUMMARY OF THE INVENTION

As a starting point, the inventors transformed *Arabidopsis thaliana* (mouse-ear cress) with the wild-type forms of the H⁺-ATPases *PMA4* or *PMA2* of *Nicotiana plumbaginifolia* (Tex-Mex tobacco). In contrast to the transformation of *Nicotiana tabacum* (cultivated tobacco) using the same constructs, the transformants of *Arabidopsis thaliana* showed a surprisingly different phenotype, in particular the *Arabidopsis thaliana* transgenic plants were characterized by demonstrably larger roots and leaves.

The invention thus provides a method for producing a transgenic plant having increased size or containing one or more organs or tissues having increased size, comprising (i) transformation of a plant with a genetic construct comprising a nucleic acid encoding a H⁺-ATPase from another species (ii) screening transformants expressing the nucleic acid for those having increased size or containing one or more organs or tissues having increased size.
The inventors have observed that transformants displaying increased (organ) size may be particularly those in which endogenous mechanisms do not down-regulate, counteract or suppress the expression of the supplied H'-ATPase from the species. Without limitation, one can envisage that such endogenous mechanisms could otherwise act upon transcription, post transcription, translation and/or post translation, such as to reduce the level and/or activity of the transformed H⁺-ATPase. Consequently, selecting an H⁺-ATPase such that it is not down-regulated, counteracted or suppressed by such endogenous mechanisms particularly refers to situations wherein the level and/or activity of a transformed H⁺-ATPase in a transformant is not reduced or is not substantially reduced, i.e., is not reduced to an extent which would preclude the H⁺-ATPase from achieving the phenotypic features in the transformant as intended by the present specification. Hence, the present invention may also be defined as a method for producing a transgenic plant having increased size or containing one or more organs or tissues having increased size, comprising (i) transformation of a plant with a genetic construct comprising a nucleic acid encoding a H⁺-ATPase from another species (ii) screening transformants expressing the nucleic acid for, wherein the expressed H⁺-ATPase is not down-regulated by an endogenous mechanism in said transformants.

By means of example and with no limitation, the expressed heterologous H⁺-ATPase may be considered not down-regulated by an endogenous mechanism in a transformed plant when the heterologous H⁺-ATPase is expressed at a level which is at least about 20%, e.g., at least about 30% or at least about 40%, preferably at least about 50%, e.g., at least about 60% or at least about 70%, more preferably at least about 80%, e.g., at least about 90% or at least about 100%, or more than 100% relative to the expression level of one or more endogenous H⁺-ATPase(s) in the transformed plant (e.g., one isoform or more or all isoforms together). Routine methods of protein quantification (e.g., protein electrophoresis and quantitative immunochemistry, or mass spectrometry quantification of signature peptides, etc.) can be employed to determine such relative relationship.

Said increase in plant or plant organ or tissue size may result in particular from increased cell elongation (enlargement of cells accompanied by changes in cell shape) and/or from increased cell proliferation and thus cell number in said plant or plant organ or tissue (e.g., in one or more tissues comprised in a plant organ). Preferably, said increase in plant or plant organ size, such as particularly increase in size of roots, leaves or hypocotyls, may result mainly or even exclusively from increased cell elongation.

In anyone of the methods as defined herein the nucleic acid may preferably encode a H⁺-ATPase from another plant species.

Whereas a transgenic plant as intended herein is produced by transformation with a nucleic acid encoding a H⁺-ATPase from another species, i.e., from a species which is not the same but is different from the plant to be transformed (the H⁺-ATPase may be conveniently denoted as heterologous with respect to the plant to be transformed therewith), the following situations may preferably apply: 1) the nucleic acid may encode H⁺-ATPase from a different species but from the same genus as the plant to be transformed therewith; or more preferably, 2) the nucleic acid may encode H⁺-ATPase from a species from a different genus than the plant to be transformed therewith. In situation 2) the different genera may belong to the same or different families, orders, classes and/or phyla. For example, in situation 2) the nucleic acid encoding the H⁺-ATPase may be from different genera but belonging to the same family, or from different families but belonging to the same order, or from different orders but belonging to the same class, or from different classes but belonging to the same phylum as the plant to be transformed therewith; or the nucleic acid encoding the H⁺-ATPase may be from a different phylum than the plant to be transformed therewith.

By preference but without limitation, the nucleic acid may encode H⁺-ATPase from a species from a plant family selected from the group consisting of *Arecaceae, Asteraceae, Brassicaceae, Cucurbitaceae, Euphorbiaceae, Fabaceae, Malvaceae, Piperaceae, Poaceae, Rubiaceae, Salicaceae, Solanaceae* and *Theaceae;* or more particularly from species from a plant genus selected from the group consisting of *Arabidopsis, Arachis, Brassica, Avena, Camellia, Cocos, Coffea, Cucumis, Elaeis, Glycine, Gossypium, Helianthus, Hordeum, Lactuca, Manihot, Medicago, Nicotiana, Oryza, Piper, Pisum, Saccharum, Salix, Secale, Solanum, Sorghum, Tagetes, Theobroma, Triticosecale, Triticum, Vicia,* and Zea; or even more particularly from a species selected from the group consisting of *Arabidopsis thaliana, Arachis hypogaea, Avena sativa, Brassica napus, Camellia sinensis, Cocos nucifera, Cucumis sativus, Glycine max, Helianthus annuus, Hordeum vulgare, Lactuca sativa, Medicago sativa, Nicotiana plumbaginifolia, Nicotiana tabacum, Oryza sativa, Piper nigrum, Pisum sativum, Secale cereale, Solanum lycopersicum, Solanum melongena, Solanum tuberosum, Theobroma cacao* and Zea *mays.*

Also preferably, the nucleic acid may encode H⁺-ATPase from a species from *Angiosperms,* such as from a species from *Eudicots,* more particularly from a species from *Asterids,* more preferably from a species from the order *Solanales,* even more preferably from a species from the family *Solanaceae,* yet more preferably from a species from the subfamily *Nicotianoideae,* and still more preferably from a species from the genus *Nicotiana,* such as particularly preferably from the species *Nicotiana plumbaginifolia.* In particular, said nucleic acid may encode a plasma membrane H⁺-ATPase of *Nicotiana plumbaginifolia,* more preferably may encode isoform 2 (PMA2) or isoform 4 (PMA4) of the plasma membrane H⁺-ATPase of *Nicotiana plumbaginifolia.*

Also preferably, the nucleic acid may encode H⁺-ATPase from a species from *Angiosperms,* such as from a species from *Eudicots,* more particularly from a species from *Rosids,* more preferably from a species from the order *Brassicales,* even more preferably from a species from the family *Brassicaceae,* yet more preferably from a species from the genus *Arabidopsis,* such as particularly preferably from the species *Arabidopsis thaliana.*

By preference but without limitation, the plant to be transformed (with a genetic construct comprising a nucleic acid encoding a H⁺-ATPase from another species) may be a species from a plant family selected from the group consisting of *Arecaceae, Asteraceae, Brassicaceae, Cucurbitaceae, Euphorbiaceae, Fabaceae, Malvaceae, Piperaceae, Poaceae, Rubiaceae, Salicaceae, Solanaceae* and *Theaceae;* or more particularly a species from a plant genus selected from the group consisting of *Arabidopsis, Arachis, Brassica, Avena, Camellia, Cocos, Coffea, Cucumis, Elaeis, Glycine, Gossypium, Helianthus, Hordeum, Lactuca, Manihot, Medicago, Nicotiana, Oryza, Piper, Pisum, Saccharum, Salix, Secale, Solanum, Sorghum, Tagetes, Theobroma, Triticosecale, Triticum, Vicia,* and Zea; or even more particularly a species selected from the group consisting of *Arabidopsis thaliana, Arachis hypogaea, Avena sativa, Brassica napus, Camellia sinensis, Cocos nucifera, Cucumis sativus, Glycine max, Helianthus annuus, Hordeum vulgare, Lactuca sativa, Medicago sativa, Nicotiana plumbaginifolia, Nicotiana tabacum, Oryza sativa, Piper nigrum, Pisum sativum, Secale cereale, Solanum lycopersicum, Solanum melongena, Solanum tuberosum, Theobroma cacao* and Zea *mays.*

Also preferably, the plant to be transformed (with a genetic construct comprising a nucleic acid encoding a H⁺-ATPase from another species) may be a species from *Angiosperms,* such as a species from *Eudicots,* more particularly a species from *Rosids,* more preferably a species from the order *Brassicales,* even more preferably a species from the family *Brassicaceae,* yet more preferably a species from the genus *Arabidopsis,* such as particularly preferably the species *Arabidopsis thaliana.*

Also preferably, the plant to be transformed (with a genetic construct comprising a nucleic acid encoding a H⁺-ATPase from another species) may be a species from *Angiosperms,* such as a species from *Eudicots,* more particularly a species from *Asterids,* more preferably a species from the order *Solanales,* even more preferably a species from the family *Solanaceae,* yet more preferably a species from the subfamily *Nicotianoideae,* and still more preferably a species from the genus *Nicotiana,* such as particularly preferably the species *Nicotiana plumbaginifolia.*

Hence, in particularly preferred embodiments the nucleic acid may encode H⁺-ATPase from a species from *Angiosperms,* such as from a species from *Eudicots,* more particularly from a species from *Asterids,* more preferably from a species from the order *Solanales,* even more preferably from a species from the family *Solanaceae,* yet more preferably from a species from the subfamily *Nicotianoideae,* and still more preferably from a species from the genus *Nicotiana,* such as particularly preferably from the species *Nicotiana plumbaginifolia,* and very preferably the nucleic acid may encode a plasma membrane H⁺-ATPase of *Nicotiana plumbaginifolia,* more preferably isoform 2 (PMA2) or isoform 4 (PMA4) of the plasma membrane H⁺-ATPase of *Nicotiana plumbaginifolia;* and the plant to be transformed with a genetic construct comprising said nucleic acid encoding a H⁺-ATPase from another species may be a species from *Angiosperms,* such as a species from *Eudicots,* more particularly a species from *Rosids,* more preferably a species from the order *Brassicales,* even more preferably a species from the family *Brassicaceae,* yet more preferably a species from the genus *Arabidopsis,* such as particularly preferably the species *Arabidopsis thaliana.*

In an alternative, the nucleic acid may encode H⁺-ATPase from a species from *Angiosperms,* such as from a species from *Eudicots,* more particularly from a species from *Rosids,* more preferably from a species from the order *Brassicales,* even more preferably from a species from the family *Brassicaceae,* yet more preferably from a species from the genus *Arabidopsis,* such as particularly preferably the species *Arabidopsis thaliana;* and the plant to be transformed with a genetic construct comprising said nucleic acid encoding a H⁺-ATPase from another species may be a species from *Angiosperms,* such as a species from *Eudicots,* more particularly a species from *Asterids,* more preferably a species from the order *Solanales,* even more preferably a species from the family *Solanaceae,* yet more preferably a species from the subfamily *Nicotianoideae,* and still more preferably a species from the genus *Nicotiana,* such as particularly preferably the species *Nicotiana plumbaginifolia.*

In embodiments, the heterologous H⁺-ATPase may display less than about 95% amino acid sequence identity to an H⁺-ATPase which is endogenous to the plant species to be transformed with said heterologous H⁺-ATPase, preferably less than about 90%, more preferably less than about 85% or less than about 80%, such as preferably between about 60% and about 95%, more preferably between about 70% and about 90% and even more preferably between about 80% and about 85% amino acid identity.

In embodiments, expression of the nucleic acid encoding a H⁺-ATPase in a transformant may be constitutive or may be inducible.

In a preferred embodiment, the expression of the nucleic acid is plant tissue- or organ-specific. Alternatively, the expression of the nucleic acid may be ubiquitous (e.g., expression in several and potentially in many or even in (substantially) all plant tissues or organs).

In a particularly preferred embodiment, the nucleic acid is expressed in (e.g. expressed at least in or expressed selectively in) one or more plant organs or tissues selected from the group comprising or consisting of roots, stems, leaves, flowers, fruits, tubers and seeds.

Genetic constructs as intended herein may comprise the nucleic acid encoding a heterologous H⁺-ATPase operably linked to one or more regulatory sequences configured to effect expression of said nucleic acid in a desired plant.

Regulatory sequences may particularly encompass promoters, enhancers, transcriptional terminators (including polyadenylation signals), etc. An operable linkage is a linkage in which regulatory sequences and sequences sought to be expressed are connected in such a way as to permit said expression. For example, sequences, such as, e.g., a promoter and an open reading frame (ORF), may be said to be operably linked if the nature of the linkage between said sequences does not: (1) result in the introduction of a frame-shift mutation, (2) interfere with the ability of the promoter to direct the transcription of the ORF, (3) interfere with the ability of the ORF to be transcribed from the promoter sequence.

In one embodiment, the genetic construct comprises (i) a promoter, (ii) a nucleic acid encoding a H⁺-ATPase from another species, (iii) a terminator, wherein the promoter, terminator and the nucleic acid are operatively linked.

In embodiments, a promoter may be configured to effect constitutive expression (a constitutive promoter) or inducible expression (an inducible promoters) or tissue- or organ-specific expression (a tissue- or organ-specific promoter) of the nucleic acid encoding the heterologous H⁺-ATPase.

Genetic constructs as intended herein may further comprise an enhancer.

In a preferred embodiment the promoter is reinforced with a single or preferably double CaMV 35S promoter enhancer.

In an embodiment, the promoter may be the PMA4 promoter from *Nicotiana plumbaginifolia,* preferably reinforced with a double CaMV 35S promoter enhancer.

A skilled person is well-aware of techniques suitable for plant transformation. Preferred methods include, for instance, *Agrobacterium-mediated* transformation (Deblaere et al. 1987. Meth Enzymol 143: 277); particle-accelerated or "gene gun" transformation (Klein et al. 1987. Nature 327: 70-73; US 4,945,050); direct microinjection into plant cells using micropipette (Crossway. 1985. Mol Gen Genetics 202: 179-185); gene transfer induced by polyethylene glycol (Krens et al. 1982. Nature 296: 72-74); fusion of plant protoplasts with other fusible, lipid-surfaced bodies (mini-cells, cells, liposomes, etc.), where the latter bodies contain the nucleic acids whose introduction is desired (Fraley et al. 1982. PNAS 79: 1859-1863); or electroporation of plant protoplasts with the to-be-introduced nucleic acid (Fromm et al. 1985. PNAS 82: 5824).

Hence, without limitation in anyone of the methods as defined herein the genetic construct may be present in a plant transformation vector, such as a plant transformation vector configured for *Agrobacterium-mediated* transformation.

In anyone of the methods as defined herein the plant is selected from the group consisting of or comprising maize, wheat, rye, oat, triticale, rice, barley, soybean, peanut, corn, cotton, rapeseed, canola, manihot, pepper, sunflower, tagetes, Solanaceous plants, potato, eggplant, tomato, Vicia species, pea, alfalfa, sorghum, cucumber, lettuce, turf grass, ornamental coffee, cacao, tea, Salix species, oil palm coconut, perennial grass, *Arabidopsis thaliana,* tobacco, ornamental plants and a forage crop.

The invention further provides a genetic construct comprising a nucleic acid encoding a H⁺-ATPase from a first species operably linked to one or more regulatory sequences configured to effect expression of said nucleic acid in a plant of a second species, wherein said first and second species are different, and wherein expression of the H⁺-ATPase in said plant is able to increase the size of said plant or of one or more of its organs. As explained above, the genetic construct may be particularly configured such that the expression of the H⁺-ATPase is not down-regulated by an endogenous mechanism in said plant.

In a preferred embodiment, the construct further comprises a promoter, wherein the promoter and nucleic acid are operatively linked. For example, the construct may further comprise a promoter and a terminator, and optionally and preferably an enhancer, wherein the promoter, terminator and nucleic acid, and if present the enhancer, are operatively linked.

In embodiments, a promoter may be configured to effect constitutive expression (a constitutive promoter) or inducible expression (an inducible promoter) or tissue- or organ-specific expression (a tissue- or organ-specific promoter) of the nucleic acid encoding the heterologous H⁺-ATPase.

In a particularly preferred embodiment the promoter is reinforced with a single or double CaMV 35S promoter enhancer.

In an even more particularly preferred embodiment the promoter is the PMA4 transcription promoter from *Nicotiana plumbaginifolia,* preferably reinforced with a double CaMV 35S promoter enhancer.

In a preferred embodiment, the nucleic acid encodes a H⁺-ATPase from *Nicotiana plumbaginifolia.*

In a particularly preferred embodiment, the nucleic acid encodes the PMA2 or the PMA4 isoform from *Nicotiana plumbaginifolia.*

By preference but without limitation, the nucleic acid may encode H⁺-ATPase from a species from a plant family selected from the group consisting of *Arecaceae, Asteraceae, Brassicaceae, Cucurbitaceae, Euphorbiaceae, Fabaceae, Malvaceae, Piperaceae, Poaceae, Rubiaceae, Salicaceae, Solanaceae* and *Theaceae;* or more particularly from species from a plant genus selected from the group consisting of *Arabidopsis, Arachis, Brassica, Avena, Camellia, Cocos, Coffea, Cucumis, Elaeis, Glycine, Gossypium, Helianthus, Hordeum, Lactuca, Manihot, Medicago, Nicotiana, Oryza, Piper, Pisum, Saccharum, Salix, Secale, Solanum, Sorghum, Tagetes, Theobroma, Triticosecale, Triticum, Vicia,* and Zea; or even more particularly from a species selected from the group consisting of *Arabidopsis thaliana, Arachis hypogaea, Avena sativa, Brassica napus, Camellia sinensis, Cocos nucifera, Cucumis sativus, Glycine max, Helianthus annuus, Hordeum vulgare, Lactuca sativa, Medicago sativa, Nicotiana plumbaginifolia, Nicotiana tabacum, Oryza sativa, Piper nigrum, Pisum sativum, Secale cereale, Solanum lycopersicum, Solanum melongena, Solanum tuberosum, Theobroma cacao* and Zea *mays.*

Also preferably, the nucleic acid may encode the H⁺-ATPase from a species from *Angiosperms,* such as from a species from *Eudicots,* more particularly from a species from *Asterids,* more preferably from a species from the order *Solanales,* even more preferably from a species from the family *Solanaceae,* yet more preferably from a species from the subfamily *Nicotianoideae,* and still more preferably from a species from the genus *Nicotiana,* such as particularly preferably from the species *Nicotiana plumbaginifolia.* In particular, said nucleic acid may encode a plasma membrane H⁺-ATPase of *Nicotiana plumbaginifolia,* more preferably may encode isoform 2 (PMA2) or isoform 4 (PMA4) of the plasma membrane H⁺-ATPase of *Nicotiana plumbaginifolia.*

By preference but without limitation, the one or more regulatory sequences may be configured to effect expression of said nucleic acid in a plant of a species from a plant family selected from the group consisting of *Arecaceae, Asteraceae, Brassicaceae, Cucurbitaceae, Euphorbiaceae, Fabaceae, Malvaceae, Piperaceae, Poaceae, Rubiaceae, Salicaceae, Solanaceae* and *Theaceae;* or more particularly of a species from a plant genus selected from the group consisting of *Arabidopsis, Arachis, Brassica, Avena, Camellia, Cocos, Coffea, Cucumis, Elaeis, Glycine, Gossypium, Helianthus, Hordeum, Lactuca, Manihot, Medicago, Nicotiana, Oryza, Piper, Pisum, Saccharum, Salix, Secale, Solanum, Sorghum, Tagetes, Theobroma, Triticosecale, Triticum, Vicia,* and Zea; or even more particularly of a species selected from the group consisting of *Arabidopsis thaliana, Arachis hypogaea, Avena sativa, Brassica napus, Camellia sinensis, Cocos nucifera, Cucumis sativus, Glycine max, Helianthus annuus, Hordeum vulgare, Lactuca sativa, Medicago sativa, Nicotiana plumbaginifolia, Nicotiana tabacum, Oryza sativa, Piper nigrum, Pisum sativum, Secale cereale, Solanum lycopersicum, Solanum melongena, Solanum tuberosum, Theobroma cacao* and *Zea mays.*

Also preferably, the one or more regulatory sequences may be configured to effect expression of said nucleic acid in a plant of a species from *Angiosperms,* such as a species from *Eudicots,* more particularly a species from *Rosids,* more preferably a species from the order *Brassicales,* even more preferably a species from the family *Brassicaceae,* yet more preferably a species from the genus *Arabidopsis,* such as particularly preferably the species *Arabidopsis thaliana.*

The invention further provides a vector comprising the genetic construct.

In a preferred embodiment, the vector is a plant transformation vector.

The invention also provides a composition configured for plant transformation comprising said genetic construct or vector.

The invention further provides the use of anyone of the genetic constructs or vectors as defined herein for the transformation of a plant, particularly for increasing the size of the plant or of one or more organs or tissues of said plant.

The invention further provides a transgenic plant obtainable by or obtained directly by anyone of the methods as defined herein, particularly wherein the transgenic plant comprises the genetic construct or vector as defined herein, more preferably wherein the transgenic plant has increased size or comprises one or more organs or tissues having increased size.

Hence, the invention also provides a transgenic plant comprising (i.e., transformed with) the genetic construct or vector as defined herein, more preferably wherein the transgenic plant has increased size or comprises one or more organs or tissues having increased size.

The invention further provides the harvestable parts and propagation material of a transgenic plant as defined herein, particularly wherein said harvestable parts or propagation material of the transgenic plant comprise the genetic construct or vector as defined herein.

Transgenic plants as intended herein may have increased size or may comprise one or more organs or tissues having increased size. Without limitation, organs having so-increased size may include roots, stems, leaves, flowers, fruits, tubers and/or seeds. Non-limiting advantages of increased size of such organs include:
- longer roots may aid the plant to acquire nutrients and water from the soil, and/or may better secure the plant in the soil thereby rendering it more resistant to uprooting;
- longer stems may allow for taller plants, and/or may increase yield where such stems are used as food or feed or for extraction of substances produced in stems (e.g., sugar cane);
- larger leaves may allow for increased photosynthesis and thus better growth and yield of plants and various organs thereof, and/or may increase yield where such leaves are used as food or feed or for extraction of substances produced in leaves, and/or may allow for aesthetic improvement, e.g., for ornamental plants;
- larger fruits, tubers or seeds may increase yield where such fruits, tubers or seeds are used as food or feed;
- larger plants any organs thereof may increase provide more raw material for bioconversion.

### BRIEF DESCRIPTION OF THE DRAWINGS

**FIG. 1** : Scheme of the construction of the plasmids.
**FIG. 2** : Screening of the homozygous F₃ plants constitutively expressing PMAs by immunodetection. 20 µg of microsomal fraction was submitted to Western blotting using specific antibodies: anti-6-His, anti-PMA4 and anti-PMA2 (to check the transgene expression) and anti-H⁺-ATPase (to identify the entire population of H⁺-ATPases).
Microsomal fraction from the wild type Arabidopsis was used as a negative control (WT) and from the wild type tobacco BY2 cells as a positive control, except for the His₆-tag detection (BY2 WT). 1 µg of purified yeast-expressed 6-His-PMA2 was used as an additional positive control (C+ yeast PMA2).
**FIG. 3** : Root growth rate of WT and the F₂ transgenic plants grown at pH 5.8.
**FIG. 4** : WT and the F₂ transgenic plants grown *in vitro.*
**FIG. 5** : Weight of WT and the F₃ transgenic plants grown at pH 5.8. 22 plants of each, WT and the F₃ transgenic plants, were planted on standard MS medium (pH 5.8) and then harvested about 4 weeks after sowing. The roots were cut off and the weight of the rosettes taken. Then they were dried at 80°C for 24h and the weight of the dried plants noted. Fig. 5A: Average leaf fresh weight of a single plant. Fig. 5B: Average leaf dry weight of a single plant.
**FIG. 6** : Hypocotyl growth rate of WT and the F₃ transgenic plants grown at pH 6.8. 30 plants of each line, WT and the F₃ transgenic plants, were grown on vertical plates containing standard MS medium (pH 6.8) in the dark. Fig. 6A: The length of the hypocotyls was measured at day 3 after germination and then the growth rate was calculated. Fig. 6B: Hypocotyl length of the F₃ transgenic plants was compared versus WT plants (ratio as %).

### DETAILED DESCRIPTION OF THE PREFERRED EMBODIMENTS

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The term also encompasses "consisting of" and "consisting essentially of".

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1 % or less, and still more preferably +/-0.1 % or less of and from the specified value, insofar such variations are appropriate to perform in the disclosed invention. It is to be understood that the value to which the modifier "about" refers is itself also specifically, and preferably, disclosed.

All documents cited in the present specification are hereby incorporated by reference in their entirety. In particular, the teachings of all documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention. When specific terms are defined in connection with a particular aspect or embodiment, such connotation is meant to apply throughout this specification, i.e., also in the context of other aspects or embodiments, unless otherwise defined.

Increase in the size of a plant, plant organ or plant tissue as used herein relates to enlargement of said plant, plant organ or plant tissue, which may result in particular from increased cell size and/or increased cell proliferation and thus cell number in said plant, plant organ or plant tissue.

Increase in cell elongation as used herein particularly relates to an enlargement of the cell, which is accompanied by changes of the cell shape and which results in an increase of the biomass production without cell proliferation.

The term "plant" is used herein in its broadest sense. It includes, but is not limited to, any species of woody, herbaceous, perennial or annual plants. It also refers to a plurality of plant cells that are largely differentiated into a structure that is present at any stage of a plant's development. Such structures include, but are not limited to, a root, stem, shoot, leaf, flower, petal, fruit, etc.

Any suitable plant can be used to produce the transgenic plants of the present invention. Non-limiting examples include tobacco, maize, wheat, rye, oat, triticale, rice, barley, soybean, peanut, corn, cotton, rapeseed, canola, manihot, pepper, sunflower, tagetes, Solanaceous plants, potato, eggplant, tomato, Vicia species, pea, alfalfa, sorghum, cucumber, lettuce, turf grass, ornamental coffee, cacao, tea, Salix species, oil palm coconut, perennial grass and a forage crop.

With "species" is meant in general a group of organisms capable of interbreeding and producing fertile offspring of both sexes (except in the case of asexually reproducing species), often sharing similarity of DNA or morphology.

The term "transgenic" when used in reference to a plant (i.e., a "transgenic plant") refers to a plant that contains at least one heterologous gene in one or more of its cells.

The term "transformants" includes the primary transformed cell and cultures derived from that cell without regard to the number of transfers. Once the introduced nucleic acid has been integrated into the genome of the plant cell, it usually is stable there and is also contained in the progenies of the originally transformed cell. All progeny may not be precisely identical in DNA content, due to deliberate or inadvertent mutations. Mutant progeny that have the same functionality as screened for in the originally transformed cell are included in the definition of transformants. Two or more generations should commonly be cultivated to make sure that the phenotypic feature is maintained stably and is transmitted. The resulting plants can be cultured normally. Seeds can be obtained from sexually reproducing plants. Sterile plants may be subjected to vegetative propagation. Techniques for the transformation of plants are known to the person skilled in the art. For example, Agrobacterium mediated plant transformation, biolistic methods like particle bombardment (Wan and Lemaux, 1994, Plant Physiol 104, 37-48; Vasil et al., 1993, Bio/Technology 11, 1553-1558; Ritala et al., 1994, Plant Mol Biol 24, 317-325; Spencer et al., 1990, Theor Appl Genet 79, 625-631), microparticle bombardment (e.g., US4945050; US5100792), protoplast transformation, gene transfer into pollen, injection into reproductive organs and injection into immature embryos can be used. Other techniques include electroporation to tissues, cells and protoplasts, protoplast fusion, and high velocity projectile introduction to cells, tissues, calli, immature and mature embryos. The choice of technique will depend largely on the type of plant to be transformed. The exogenous nucleic acid can be introduced into any suitable cell(s) of the plant, such a root cell(s), stem cell(s) and/or leaf cell(s) of the plant.

The use of agrobacteria for the transformation of plant cells has extensively been examined and sufficiently disclosed in the specification of EP120516, in Hoekema (1985, In: The Binary Plant Vector System, Offsetdrukkerij Kanters B. V., Alblasserdam, Chapter V), Fraley et al., 1986, Crit Rev Plant Sci 4, 1-46 and An et al., 1985, EMBO J 4, 277-287. Plant explants can be co-cultivated with *Agrobacterium tumefaciens* or *Agrobacterium rhizogenes.* From the infected plant material (for example leaf explants, segments of stems, roots but also protoplasts or suspension cultivated plant cells) whole plants can be regenerated in a suitable medium which may contain antibiotics or biozides for the selection of transformed cells.

The term "nucleic acid" as used herein particularly encompasses RNA or DNA that is linear or branched, single or double stranded, or a hybrid thereof. The term also encompasses RNA/DNA hybrids.

The terms "genetic construct" and "construct" are used interchangeably herein and refer to an artificially assembled nucleic acid which includes the gene or genes of interest. In general a genetic construct comprises the gene or genes of interest and appropriate regulatory sequences. In a preferred embodiment, the construct may include regulatory elements such as a promoter and an enhancer that control or influence the transcription of the nucleic acid according to the present invention; said regulatory elements and nucleic acid being operatively linked. By "operatively linked", as used herein, is meant that said regulatory elements are capable of causing expression of said nucleic acid in a plant cell. Techniques for operatively linking the components of the genetic construct of the present invention are well known to those skilled in the art. Such techniques include the use of linkers, such as synthetic linkers, for example including one or more restriction enzyme sites.

The term "promoter" as used herein, refers to a DNA sequence that is located at the 5' end (i.e. precedes the gene of interest). The promoter functions as a switch, activating the expression of a gene. If the gene is activated, it is said to be transcribed. Transcription involves the synthesis of mRNA from the gene. The promoter, therefore, serves as a transcriptional regulatory element and also provides a site for initiation of transcription of the gene into mRNA.

A variety of promoters which may be employed in the genetic constructs of the present invention are well known to those skilled in the art. Factors influencing the choice of promoter include the desired tissue specificity of the expression, and whether constitutive or inducible expression is desired and the nature of the plant cell to be transformed.

In the context of the present invention "tissue-specific expression" indicates that the transgene will only be expressed in particular organs or tissues where the transgene product is desired, leaving the rest of the organs or tissues in the plant unmodified by transgene expression. Non exhaustive examples are promoters specific for roots, tubers, fruits, stems, leafs, fruits, seed, etc.

For example, plant root-specific promoters may include *inter alia* those in the following table:

| **Gene Source** | **Reference** |
|---|---|
| Rice RCc3 | Xu et al (1995) Plant Mol Biol 27(2): 237-48 |
| Arabidopsis phosphate transporter PHT1 | Kovama *et al.,* 2005 |
| Medicago phosphate transporter | Xiao *et al.,* 2006 |
| Arabidopsis Pyk10 | Nitz et al. (2001) Plant Sci 161 (2): 337-346 |
| Tobacco root-specific genes RB7, RD2, RD5, RH12 | Conkling et al. (1990) Plant Phys 93(3): 1203-1211 |
| Barley root-specific lectin | Lerner & Raikhel (1989) Plant Phys 91: 124-129 |
| Root-specific hydroxy-proline rich protein | Keller & Lamb (1989) Genes & Dev 3:1639-1646 |
| Arabidopsis CDC27B/hobbit | Blilou et al. (2002) Genes & Dev 16:2566-2575 |

Other organ- or tissue-specific promoters are generally available to a skilled person.

With "constitutive expression" is meant herein that a gene is transcribed at constant level compared to "inducible expression", with which is meant a gene that is only transcribed when needed. Particularly suitable constitutive promoters include the Cauliflower Mosaic Virus 35S (CaMV 35S) promoter and derivatives thereof, the maize Ubiquitin promoter, and the rice Actin promoter.

Examples of plant constitutive promoters include *inter alia* those in the following table:

| **Gene Source** | **Reference** |
|---|---|
| Actin | McElroy et al, Plant Cell, 2: 163-171, 1990 |
| HMGB | WO 2004/070039 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| Rubisco small subunit | US 4,962,028 |
| ocs | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| V-ATPase | WO 01/14572 |
| G-box proteins | WO 94/12015 |

The term "enhancer" refers to a short region of DNA that can increase transcription of genes upon binding of activator proteins. In a preferred embodiment the Cauliflower Mosaic Virus 35S (CaMV 35S) enhancer is used.

The term "terminator" refers to a sequence element at the end of a transcriptional unit which signals termination of transcription. Terminators active in plant cells are known and well-documented in the literature, such as, e.g. the nopaline synthase (NOS) gene terminator of *Agrobacterium tumefaciens,* the terminator of the Cauliflower mosaic virus CaMV35S gene, the zein gene terminator from *Zea mays,* the ribulose-1,5-biphosphate carboxylase small subunit gene (rbcS la) terminator, and the isopentenyladenine transferase (ipt) terminator, amongst others.

The term "vector" as used herein encompasses both expression and transformation vectors. Preferably the vector is a plant transformation vector. Vectors are often recombinant molecules containing nucleic acids from several sources. In the present invention a vector comprises the genetic construct as defined herein. Preferably, the vectors of the invention additionally comprise a selectable marker gene.

Selectable marker genes are well known to those skilled in the art and comprise, for example, antimetabolite resistance as the basis of selection for dhfr, which confers resistance to methotrexate (Reiss, 1994, Plant Physiol (Life Sci Adv) 13, 143-149) ; npt, which confers resistance to the aminoglycosides neomycin, kanamycin and paromycin (Herrera-Estrella, 1983, EMBO J 2, 987-995) and hygro, which confers resistance to hygromycin (Marsh, 1984, Gene 32, 481-485). Additional selectable genes have been described, namely trpB, which allows cells to utilize indole in place of tryptophan ; hisD, which allows cells to utilize histinol in place of histidine (Hartman, 1988, Proc Natl Acad Sci USA 85, 8047) ; mannose-6-phosphate isomerase which allows cells to utilize mannose (WO199420627) and ODC (ornithine decarboxylase) which confers resistance to the ornithine decarboxylase inhibitor, 2-(difluoromethyl)-DL-ornithine, DFMO (McConlogue, 1987, In : Current Communications in Molecular Biology, Cold Spring Harbor Laboratory ed.) or deaminase from Aspergillus terreus which. confers resistance to Blasticidin S (Tamura et al., 1995, Biosci Biotechnol Biochem 59, 2336- 2338).

Following transformation, transformed cells containing the introduced nucleic acid can be selected from cells lacking the introduced nucleic acid using routine selection and/or testing techniques. Selectable markers on the genetic construct or vector are particularly useful for simple and rapid screening and selection of transformants expressing the nucleic acid of the invention. Alternatively, the expression of the introduced nucleic acid may be screened by conventional methods known to a person skilled in the art, for example by extracting proteins from the plasma membrane of the transgenic plants and testing with antibodies directed against the protein encoded by the nucleic acid.

The term "H⁺-ATPase" refers to a proton-pumping ATPase that is present in the plasma membrane of plant cells, where it couples hydrolysis of ATP with proton transport outside the cell. The present invention particularly relates to H⁺-ATPase molecules obtainable from plants. The term H⁺-ATPase, as used herein, also refers to mutants (deletions, additions, point mutations) that result in a modification of regulation or activity, such as particularly wherein only the cytoplasmic portion of the plasma membrane H⁺-ATPase is retained. Also comprised by the present disclosure are chimeric molecules which contain portions of amino acid residues derived from more than one H⁺-ATPase. Likewise, the H⁺-ATPase molecules may be obtained from one species or be composed of sequences derived from two or more species. The present disclosure also encompasses modified H⁺-ATPase molecules, such as, for example addition of histidine residue ("his-tag") or of other tags such as for example HA-tag, Strep-tag, Flag-tag or a Myc-tag. The person skilled in the art knows various techniques of generating chimeric or hybrid nucleic acid molecules which encode chimeric or hybrid protein molecules.

Sequence identity may be determined using suitable algorithms for performing sequence alignments and determination of sequence identity as know *per se.* Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, e.g., for the BLASTN algorithm: cost to open a gap = 5, cost to extend a gap = 2, penalty for a mismatch = -2, reward for a match = 1, gap x_dropoff = 50, expectation value = 10.0, word size = 28; or for the BLASTP algorithm: matrix = Blosum62, cost to open a gap = 11, cost to extend a gap = 1, expectation value = 10.0, word size = 3).

In yet another aspect, the invention also relates to harvestable parts and to propagation material of the transgenic plants according to the invention. Harvestable parts can be in principle any useful parts of a plant, such as for example, leaves, stems, flowers, fruit, seeds, roots etc. Propagation material includes, but is not limited to, seeds, fruits, cuttings, seedlings, tubers, rootstocks etc.

The H⁺-ATPase gene used in the present invention can be selected from other plants such as: *Arachis hypogaea, Avena sativa, Brassica napus, Camellia sinensis, Cocos nucifera, Cucumis sativus, Glycine max, Helianthus annuus, Hordeum vulgare, Lactuca sativa, Medicago sativa, Nicotiana tabacum, Oryza sativa, Piper nigrum, Pisum sativum, Secale cereale, Solanum lycopersicum, Solanum melongena, Solanum tuberosum, Theobroma* cacao and Zea *mays* can be introduced in any suitable plant.

### EXAMPLES

The invention is illustrated by the following non-limiting examples

### Material and methods

### Antibodies

PMA2 and PMA4 that were expressed in Arabidopsis were provided with a 6-His tag. In order to follow their expression, anti-His antibodies were used (Penta-His, QIAGEN). To follow PMA2 and PMA4 expression, regardless of the 6-His tag and presence of Arabidopsis H⁺-ATPases, isoform-specific polyclonal antibodies raised against peptides specific for either PMA2 (1A7, 5A7) or PMA4 (2A8 or 6A8) were used (Moriau et al., 1999, Plant J 19, 31-41). The total amount of all H⁺-ATPases was checked thanks to the pan PMA antibodies (W1 C, Table 1) (Morsomme et al., 1998, J Biol Chem 273, 34837-34842).

**Table 1. Representation of antibodies used in the experiments.**

| **1° Antibody** | **Target Protein** | **Target Sequence** | **2° Antibody** | **References** |
|---|---|---|---|---|
| 1A7 | PMA2 | 437AHNKSDIERR466 | anti-rabbit | Moriau *et al.,* 1999 |
| 2A8 | PMA4 | 436CNAKEDVRRK465 | anti-rabbit | Moriau *et al.,* 1999 |
| 5C7 | PMA2 | 905TNFELNQLAE915 | anti-rabbit | Moriau *et al.,* 1999 |
| 6A8 | PMA4 | 901 NSYRELSEIAE911 | anti-rabbit | Moriau *et al.,* 1999 |
| W1C | pan PMA | whole protein | anti-rabbit | Morsomme et al., 1998 |
| Penta-His | 6-His | 5-His | anti-mouse | QIAGEN |

### Bacteria

*Agrobacterium tumefaciens* GV3101::pMP90 (Koncz and Schell, 1986, Mol Gen Genet 204, 383-396) or LBA4404.pBBR 1MCS-5.VirGN54D (van der Fits et al., 2000, Plant Mol Biol 43, 495-502) were used for Arabidopsis transformation. Both were grown at 28°C in 2YT medium (1.6% tryptone, 1 % yeast extract, 0.5% NaCl, 0.2% glucose, 0.04% MgS04, 0.8% agar for solid medium) supplemented with 40 mg/l gentamycin, 20 mg/l rifampicin and 100 mg/l spectinomycin, whenever carrying one of the pMODUL-X plasmids. *Escherichia coli* JM109 was used as the host strain during construction of the plasmids and their amplification (Yanisch-Perron et al., 1985, Gene 33, 103-119).

### Plants

*Arabidopsis thaliana* ecotype Col-0 (Columbia) (Meinke and Scholl, 2003, Plant Physiol 133, 1046-1050) was used as wild type. Transgenic plants expressing 6-His-tagged PMA4 and PMA2 were obtained using Agrobacterium-mediated floral dip method. Plants were grown in soil in a growth chamber at 21 °C, with a 8-16 h photoperiod (-185 µmol m-2s-1) and 60-80% humidity, depending on the conditions chosen.
Plants grown *in vitro* were kept on Murashige & Skoog medium (ICN, Costa Mesa, CA), supplemented with 1% sucrose and optionally with 0.05% MES, 100 mg/l myo-inositol, 2.5 mg/l thiamine, 1 mg/l pyridoxine, 1 mg/l nicotinic acid and 0.6-0.8% agar for solid media, pH 5.8 - 8.4 (KOH). In vitro cultures were grown in a growth chamber at 21 °C, with 16 h photoperiod (-45 µmol m-2s-1 ).

### Transformation of bacteria

Either E. *coli* or *A. tumefaciens* competent cells were transformed by electroporation (Gene Pulser™, BioRad). Conditions for E. *coli* electroporation were 200 ohms, capacitance 25 µFD, voltage 1.70 kV/cm2. Conditions for *A. tumefaciens:* 400 ohms, capacitance 25 µFD, voltage 1.25 kV/cm2. Briefly, 50 µl of competent bacteria was electroporated with 0.5 - 2 µl of plasmid. 1 ml of LB medium (in case of E. *coli*) or SOC medium (2% tryptone, 0.5% yeast extract, 10 mM NaCl, 2.5 mM KCI, 10 mM MgS04, 10 mM MgCl2, 20 mM glucose) (in case of *A. tumefaciens*) were added and bacteria were incubated for 1h at 37°C, or 4h at 28°C, respectively. 1/10 to 1/2 of the cell suspension was spread on a solid medium allowing proper selection and isolation of single colonies.

### DNA handling

Isolation of DNA, restriction analysis and PCR reactions were performed according to standard protocols of molecular biology (Sambrook et al., 1989, Molecular cloning: a laboratory manual, Cold Spring Harbor Laboratory Press, Cold Spring Harbor, New York, USA).

### Microsomal fraction isolation from plant tissues

The plant material was either finely ground in liquid nitrogen or crushed directly in the homogenization buffer. The ground powder was resuspended in homogenization buffer in ratio 1 g : 2 ml (250 mM sorbitol, 60 mM Tris, 10 mM EDTA, 5 mM EGTA, 20% ethyleneglycol, 30 mM β-glycerol phosphate, 10 mM NaF, 5 mM Na2Mo04*2H20, pH 8.0 (HCl); directly before use 5 mM DTT, 1 mM phenylmethylsulfonyl fluoride, 0.6% PVP and the protease inhibitors leupeptin, aprotinin, antipain, pepstatin and chymostatin each at 2 µg/ml were added). The homogenate was centrifuged for 5 min at 1,935 x g, 4°C and the obtained supernatant was centrifuged again for 5 min at 4,355 x g, 4°C. The resulting supernatant was filtrated through Miracloth (Calbiochem) and centrifuged for 30 min at 100,000 x g, 2°C. The pellet (microsomal fraction) was resuspended in suspension buffer (250 mM sorbitol, 20% ethylene glycol, 30 mM β-glycerol phosphate, 10 mM NaF, 60 mM Tris, pH 8.0 (HCl) and the protease inhibitors leupeptin, aprotinin, antipain, pepstatin and chymostatin each at 2 µg/ml) and stored at - 80°C.

### Protein assay

Protein quantification was performed according to Bradford (1976, Annal Biochem 72, 248-254) with BSA as a standard.

### SDS-PAGE electrophoresis

Proteins were solubilized for 15 min, at room temperature in the Laemmli solubilization buffer (Laemmli (1970) Nature 227, 680-685), supplemented with 9 M urea or not (80 mM Tris, 2% SDS (w/v), 10% glycerol (w/v), 0.005% bromophenol blue (w/v), 1% DTT, (9 M urea), 1 mM phenylmethylsulfonyl fluoride and 2 µg/ml of each protease inhibitor: leupeptin, aprotinin, antipain, pepstatin and chymostatin, pH 6.8 (HCl)). Total protein extracts (25 - 30 µg), microsomal fractions (20- 30 µg) or plasma membrane fractions (10 µg) were typically analyzed by SDS-PAGE (10% polyacrylamide), according to Laemmli (1970).

### Western blotting

After SDS-PAGE, the gel was incubated for 5 min in the transfer buffer (15% (v/v) methanol, 50 mM Tris, 200 mM glycine and 0.05% (w/v) SDS). Meanwhile, a polyvinylidene fluoride(PVDF) membrane was briefly wetted in methanol and incubated for 5 min in the transfer buffer. Semi-dry transfer was performed (Trans-blot Semi Dry System, BioRad) for 50 min at 25 volts. After transfer, the blot was saturated overnight at 4°C in 3% (w/v) dried milk (Nestlé), 0.5% Tween-20 in TBS buffer (20 mM Tris, 136 mM NaCl, pH 7.4 (HCl)).

For immunodetection, the milk-saturated blot was rinsed with 0.1 % Tween-80 in TBS buffer (5 min) and then incubated for 2.5 h in the same medium supplemented with selected primary antibodies. After three 5-min washes with the same buffer without antibodies, the blot was incubated for 1.5 h in the same buffer containing secondary antibodies conjugated to the peroxidase. The blot was washed three to four times for 5 min with the same buffer and subjected to the chemiluminescent (ECL) detection with luminal-like substrate (Roche kit).

### PVDF membrane stripping

For reusing PVDF blots, these were rinsed briefly in water (5 min) and then in 0.4 N NaOH. Next, the blots were incubated for 10 min in 50 ml 0.4 N NaOH at room temperature. The blot was then washed thrice for 3 min in water.

### Example 1 Transformation of Arabidopsis plants

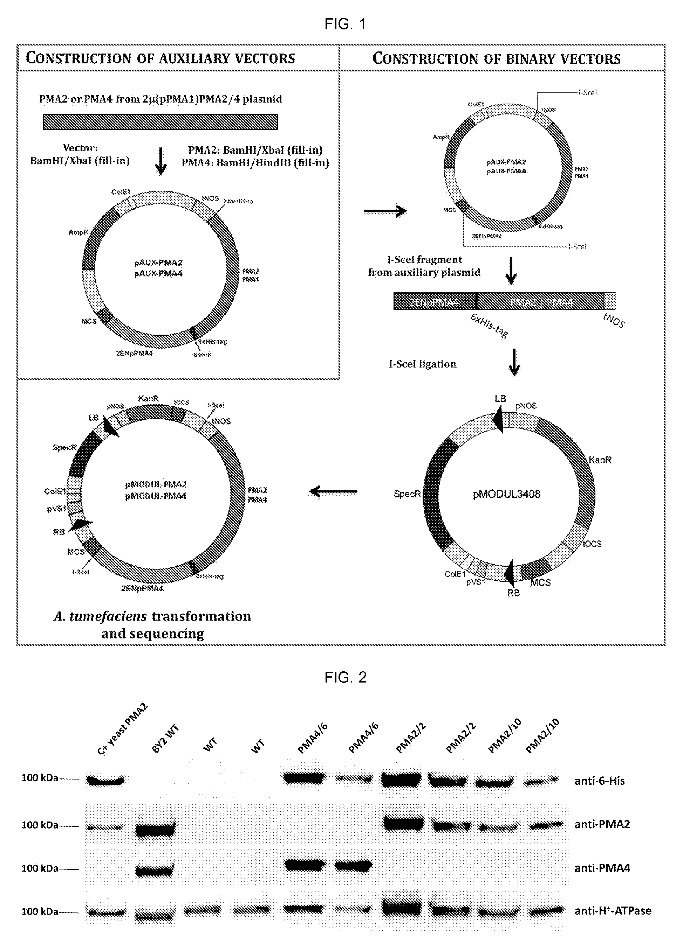

The wild type forms of *PMA4* and *PMA2* from *Nicotiana plumbaginifolia* were tagged with 6 His codons in the N-terminal coding region. 6 His codons were inserted between the 2^{nd} and 3^{rd} codon of *PMA4* and between the 3^{rd} and 4^{th} codon of *PMA2.* These genes were put under the control of the *PMA4* promoter reinforced with a 165-bp CaMV 35S enhancer (-209 to -45 upstream of the 35S transcription start) at two positions: -50 and -500 (2ENpPMA4; Zhao et al., 1999, Plant Sci 149, 157-165), giving the constructs 2ENpPMA4-PMA4 and 2ENpPMA4-PMA2.
The plasmids pMODUL-PMA4 and pMODUL-PMA2 were created based on the pMODUL3408 binary plasmid, a member of the pPZP family (Hajdukiewicz et al., 1994, Plant Mol Biol 25, 989-994; Goderis et al., 2002, Plant Mol Biol 50, 17-27; Table 1; Figure 1). All constructs have a spectinomycin cassette for the selection in *Agrobacterium tumefaciens* and a kanamycin cassette for the selection in the plants.

After validation by sequencing the constructs were used to transform *Agrobacterium tumefaciens.* Resulting strains were then checked again by sequencing of the retrieved plasmids to exclude any possibility of DNA rearrangement.

Arabidopsis plants were transformed by floral-dip, using the *A. tumefaciens* strain GV3101::pMP90 as mediator. *A. tumefaciens* was grown at 28°C on 2YT solid medium supplemented with selective antibiotics. A 50-ml liquid culture was started and grown for 36 h in 28°C on the rotation table. One ml of this culture was used to further inoculate 250 ml 2YT containing proper antibiotics and grown for another 24 h under the same conditions. Then the culture was centrifuged for 5 min at 3,830 x g (room temperature). The supernatant was removed and the pellet resuspended in 5% sucrose solution to final OD600=0.8. Right before use, Silwet L-77 (OSi Specialties Inc.) was added to a final concentration of 0.02%. The suspension of Agrobacterium was poured into a pyrex bowl and used for floral-dip of the Arabidopsis. Only plants with majority of flower buds and little actual flowering were chosen for transformation. Plants were dipped in the bacterial suspension twice for 1 min and then they were left lying on the side for 24 h under cover of a plastic bag to prevent drying of the film that formed on the plants.

After 3-4 weeks seeds were collected and were then sterilized (washing 1 min 70% ethanol, 20 min 1/2 bleach (Loda) with 0.01% Triton X-100 (Sigma), and four times sterile water) and plated onto selective MS media, supplemented with 400 mg/l carbenicillin, 50 mg/l nystatin and either 50 mg/l kanamycin or 25 mg/l hygromycin. The resistant plants were then transferred into soil and selection of positive transformants was performed on a kanamycin medium and through detection of the His₆-tag of the introduced PMAs or using specific anti-PMA2 and anti-PMA4 antibodies (Fig. 2). The presence of the transgene was also checked by PCR, using plant genomic DNA as template. We obtained 6 lines transformed with pMODUL-PMA4 construct which showed expression of the transgene (out of 25 screened on medium supplemented with 50 µg/ml kanamycin) and 9 with pMODUL-PMA2 (out of 33).

Seeds of positive transformants were again collected, giving rise to the following F2 and F3 generations plants.

### Example 2 Growth of the transgenic plants

The second generation plants (F₂) (heterozygous) were first screened on a kanamycin medium in order to eliminate WT plants.

Wild type (WT) and the F₂ transgenic plants were grown at different pH and in normal growth conditions. Measurement of the root and shoot was performed.

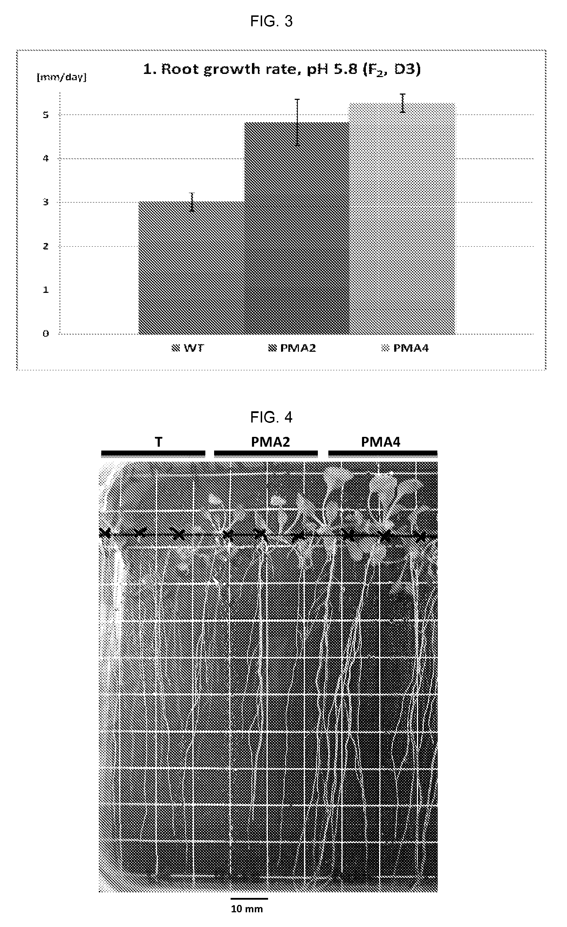

The transgenic lines showed generally faster root growth, which, compared to the WT plants, could be from 10% to 74% faster for PMA2- and PMA4-expressing Arabidopsis, depending on the tested conditions (Fig. 3). Also the size of the rosettes was different, as well as the length of the hypocotyl (Fig. 4). Conditions: ½ MS medium, 1% sucrose, F2 plants, 14 days, pH 5.8 for Fig 3 and 6.8 for Fig 4.

### Example 3 Weight of the transgenic plants

The transformed Arabidopsis plants were screened regarding the transgene expression and their homozygosity in the third generation plants (F₃), which allowed us to characterize three independent, stable transgenic lines that possess a double copy of the insert (they are homozygous) and still show transgene expression. Two of them express PMA2 (PMA2/2 and MPA2/10) and one PMA4 (PMA4/6) isoform.

22 plants of each line were planted onto standard MS medium (pH 5.8) and then harvested about four weeks after sowing. The roots were cut off and the weight of the rosettes taken. Then they were dried at 80°C for 24h and the weight of the dried plants noted.

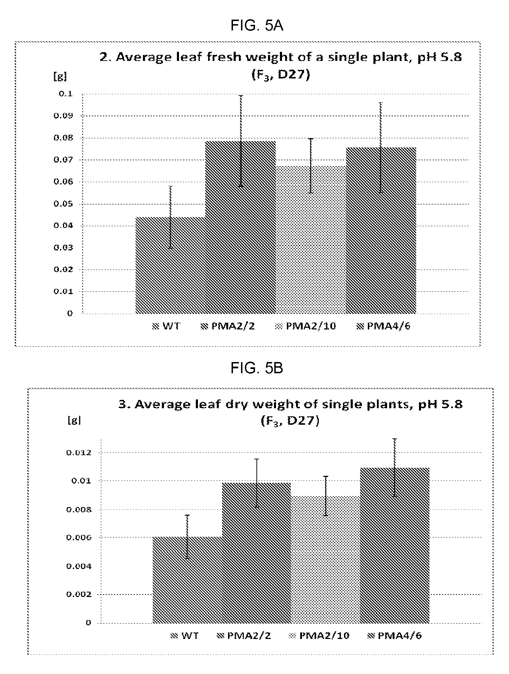

Transgenic plants grown *in vitro* showed 53-78% higher leaf fresh weight than WT plants (Fig. 5A). Also the transgenic plants showed 47-80% higher leaf dry mass compared to WT plants (Fig. 5B).

### Example 4 Length of hypocotyls of the transgenic plants

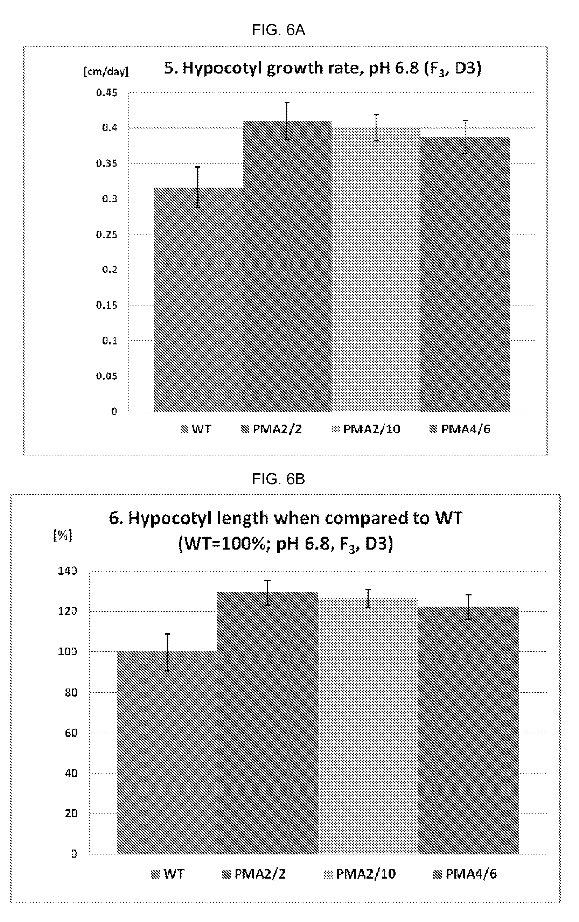

WT and the F₃ transgenic plants of example 3 were grown on vertical plates containing standard MS medium (pH 6.8) in the dark, in order to enhance the growth of the hypocotyls. Every 24h the plates were scanned, so we could follow the growth of the plants and then analyze the data with the SmartRoot program, measuring the elongation of the hypocotyls. The length of hypocotyls was measured for 30 plants of each tested line (WT, PMA2/2, PMA2/10, PMA4/6) at day 3 after germination and then the growth rate was calculated (Fig. 6A).

The hypocotyls of the transgenic lines were on average 22-29% longer when compared to WT plants (Fig. 6B).

### Example 5 reverse experiment

In order to exclude the fact the possibility that the effect on cell elongation is caused by the specific H⁺-ATPase used in the above experiments, the experiment is reversed, i.e. by using a *Arabidopsis thaliana* H⁺-ATPase (cf. Harper et al., 1990, The Journal of Biological Chemistry Col. 265(23):13601-13608) and transfect it into the *Nicotiana plumbaginifolia* plant, using the same technology as in Example 1.

### Example 6 Arabidopsis thaliana H⁺-ATPase in other plants

Using the methodology of examples 1 and 5, an H⁺-ATPase gene of *Arabidopsis thaliana* can be transfected into other plants such as: *Arachis hypogaea, Avena sativa, Brassica napus, Camellia sinensis, Cocos nucifera, Cucumis sativus, Glycine max, Helianthus annuus, Hordeum vulgare, Lactuca sativa, Medicago sativa, Nicotiana tabacum, Oryza sativa, Piper nigrum, Pisum sativum, Secale cereale, Solanum lycopersicum, Solanum melongena, Solanum tuberosum, Theobroma cacao* and Zea *mays.*

### Example 7 Nicotiana plumbaginifolia H⁺-ATPase in other plants

Using the methodology of example 1, the H⁺-ATPase gene of *Nicotiana plumbaginifolia* can be transfected into other plants such as: *Arachis hypogaea, Avena sativa, Brassica napus, Camellia sinensis, Cocos nucifera, Cucumis sativus, Glycine max, Helianthus annuus, Hordeum vulgare, Lactuca sativa, Medicago sativa, Nicotiana tabacum, Oryza sativa, Piper nigrum, Pisum sativum, Secale cereale, Solanum lycopersicum, Solanum melongena, Solanum tuberosum, Theobroma cacao* and Zea *mays.*

## Claims

1. A method for producing a transgenic plant having increased size or containing one or more organs or tissues having increased size, comprising
- transformation of a plant with a genetic construct comprising a nucleic acid encoding a H⁺-ATPase from another species
- screening transformants expressing the nucleic acid for those having increased size or containing one or more organs or tissues having increased size.

2. The method according to claim 1, wherein the expressed H⁺-ATPase is not down-regulated by an endogenous mechanism in said transformants.

3. The method according to any one of claims 1 or 2, wherein the increase in plant or plant organ or tissue size results from increased cell elongation and/or from increased cell proliferation in said plant or plant organ or tissue.

4. The method according to any one of claims 1 to 3, wherein the nucleic acid encodes H⁺-ATPase from a different species but from the same genus as the plant to be transformed therewith, more preferably wherein the nucleic acid encodes H⁺-ATPase from a species from a different genus than the plant to be transformed therewith.

5. The method according to any one of claims 1 to 4, wherein the nucleic acid encodes H'-ATPase from a plant species, particularly from a species from a plant family selected from the group consisting of *Arecaceae, Asteraceae, Brassicaceae, Cucurbitaceae, Euphorbiaceae, Fabaceae, Malvaceae, Piperaceae, Poaceae, Rubiaceae, Salicaceae, Solanaceae* and *Theaceae;* or more particularly from species from a plant genus selected from the group consisting of *Arabidopsis, Arachis, Brassica, Avena, Camellia, Cocos, Coffea, Cucumis, Elaeis, Glycine, Gossypium, Helianthus, Hordeum, Lactuca, Manihot, Medicago, Nicotiana, Oryza, Piper, Pisum, Saccharum, Salix, Secale, Solanum, Sorghum, Tagetes, Theobroma, Triticosecale, Triticum, Vicia,* and Zea; even more particularly from a species selected from the group consisting of *Arabidopsis thaliana, Arachis hypogaea, Avena sativa, Brassica napus, Camellia sinensis, Cocos nucifera, Cucumis sativus, Glycine max, Helianthus annuus, Hordeum vulgare, Lactuca sativa, Medicago sativa, Nicotiana plumbaginifolia, Nicotiana tabacum, Oryza sativa, Piper nigrum, Pisum sativum, Secale cereale, Solanum lycopersicum, Solanum melongena, Solanum tuberosum, Theobroma cacao* and *Zea mays,* or wherein the nucleic acid encodes H⁺-ATPase from a species from *Angiosperms,* such as from a species from *Eudicots,* more particularly from a species from *Asterids,* more preferably from a species from the order *Solanales,* even more preferably from a species from the family *Solanaceae,* yet more preferably from a species from the subfamily *Nicotianoideae,* and still more preferably from a species from the genus *Nicotiana,* such as particularly preferably from the species *Nicotiana plumbaginifolia;* preferably said nucleic acid encodes a plasma membrane H⁺-ATPase of *Nicotiana plumbaginifolia,* more preferably isoform 2 (PMA2) or isoform 4 (PMA4) of the plasma membrane H⁺-ATPase of *Nicotiana plumbaginifolia.*

6. The method according to any one of claims 1 to 5, wherein H⁺-ATPase displays less than about 95% amino acid sequence identity to an H⁺-ATPase which is endogenous to the plant species to be transformed, preferably less than about 90%, more preferably less than about 85% or less than about 80%, such as preferably between about 60% and about 95%, more preferably between about 70% and about 90% and even more preferably between about 80% and about 85% amino acid identity.

7. The method according to any one of claims 1 to 6, wherein the expression of the nucleic acid is plant tissue-specific, preferably wherein the nucleic acid is expressed in a plant organ or tissue selected from the group comprising roots, leaves, tubers, stems, flowers, fruits and seeds.

8. The method according to any one of claims 1 to 7, wherein the genetic construct is present in a plant transformation vector, and comprises:
- a promoter
- a nucleic acid encoding a H⁺-ATPase from another species,
- a terminator
wherein the promoter, terminator and the nucleic acid are operatively linked.

9. The method according to claim 8, wherein the promoter is configured to effect constitutive expression or inducible expression or tissue- or organ-specific expression of the nucleic acid encoding the H⁺-ATPase from another species.

10. The method according to any one of claims 1 to 9, wherein said plant is selected from the group consisting of comprising maize, wheat, rye, oat, triticale, rice, barley, soybean, peanut, corn, cotton, rapeseed, canola, manihot, pepper, sunflower, tagetes, Solanaceous plants, potato, eggplant, tomato, Vicia species, pea, alfalfa, sorghum, cucumber, lettuce, turf grass, ornamental coffee, cacao, tea, Salix species, oil palm coconut, perennial grass, *Arabidopsis thaliana,* tobacco, ornamental plants and a forage crop.

11. A genetic construct comprising a nucleic acid encoding a H⁺-ATPase from a first species operably linked to one or more regulatory sequences configured to effect expression of said nucleic acid in a plant of a second species, wherein said first and second species are different, and wherein expression of the H⁺-ATPase in said plant is able to increase the size of said plant or of one or more of its organs, said genetic construct optionally comprising a promoter, wherein the promoter and nucleic acid are operatively linked, preferably wherein the promoter is configured to effect constitutive expression or inducible expression or tissue- or organ-specific expression of the nucleic acid encoding the H⁺-ATPase in the plant, preferably wherein said promoter is the *PMA4* transcription promoter from *Nicotiana plumbaginifolia* reinforced with a double CaMV 35S promoter enhancer.

12. The construct according to claim 11, wherein the nucleic acid encodes H⁺-ATPase from a species from *Angiosperms,* such as from a species from *Eudicots,* more particularly from a species from *Asterids,* more preferably from a species from the order *Solanales,* even more preferably from a species from the family *Solanaceae,* yet more preferably from a species from the subfamily *Nicotianoideae,* and still more preferably from a species from the genus *Nicotiana,* such as particularly preferably from the species *Nicotiana plumbaginifolia;* preferably said nucleic acid encodes a plasma membrane H⁺-ATPase of *Nicotiana plumbaginifolia,* more preferably isoform 2 (PMA2) or isoform 4 (PMA4) of the plasma membrane H⁺-ATPase of *Nicotiana plumbaginifolia.*

13. A vector comprising the genetic construct according to any one of claims 11 to 12, preferably wherein said vector is a plant transformation vector.

14. Use of the genetic construct according to any one of claims 11 to 12 or the vector according to claim 13 for the transformation of a plant for increasing the size of the plant or of one or more organs or tissues of said plant.

15. A transgenic plant obtainable or directly obtained by the method of any one of claims 1 to 10, or comprising the genetic construct according to any one of claims 11 to 12 or the vector according to claim 13, or propagation material or harvestable parts of such a transgenic plant.
